# EUROPÄISCHE PATENTSCHRIFT

(11) **EP 2 316 330 B1**
(45) Veröffentlichungstag und Bekanntmachung des Hinweises auf die Patenterteilung: **28.11.2012**
(21) Anmeldenummer: 10004597.0
(22) Anmeldetag: 30.04.2010
(51) Int. Cl.: A61B 3/13, A61B 3/12, A61B 3/14, G02B 21/22, G02B 27/22, G02B 17/04, G02B 7/18, G02B 21/24, G02B 21/36, A61B 19/00

(54) **Modul zur steroskopischen Weitwinkel-Fundusbeobachtung für ein ophthalmologisches Operationsmikroskop**
Module for stereoscopic wide angle fundus observation for an ophthalmological operation microscope
Module d'observation ophtalmoscopique à grand angle stéréoscopique pour un microscope d'opération ophtalmologique

(30) Priorität: 29.10.2009 DE 202009014603 U
(43) Veröffentlichungstag der Anmeldung: 04.05.2011
(73) Patentinhaber: Möller-Wedel GmbH, 22880 Wedel (DE)
(72) Erfinder: Koetke, Jochen, 22087 Hamburg (DE); Trede, Jens, 25488 Holm (DE)
(74) Vertreter: Glawe, Delfs, Moll

(56) Entgegenhaltungen:
- EP-A1- 1 326 117
- EP-A2- 0 054 128
- WO-A1-91/15150
- DE-A1- 2 328 364
- DE-A1- 2 353 101
- DE-A1- 3 615 842
- DE-A1- 19 613 431
- DE-U1- 20 021 955
- JP-A- 2006 006 417
- JP-A- 2009 229 980
- US-A- 5 321 447

## Beschreibung

Die Erfindung betrifft ein Modul zur stereoskopischen Weitwinkel-Fundusbeobachtung für ein ophthalmologisches Operationsmikroskop der Augenchirurgie zur Positionierung zwischen einem Patientenauge und der Frontlinse des Operationsmikroskops, mit einer Opthalmoskopierlinse mit einer optischen Achse zur Abbildung des Fundus in ein Zwischenbild, mit einer Prismenanordnung zur optischen Aufrichtung des Zwischenbildes, mit einem Gehäuse, das die Anordnung aus Ophthalmoskopierlinse und Prismenanordnung enthält, und mit einer Vorrichtung zum Montieren an ein Operationsmikroskop
mit einem Drehlager zum Drehen des Moduls um die Achse des Drehlagers und einer Aufhängung für das Mikroskop.

Derartige Systeme sind aus der Patentliteratur, beispielsweise WO 91/15150, EP 1 227 355, bekannt, die Module offenbaren nach dem Oberbegriff des Anspruch 1.

Operationsmikroskope dienen der vergrößerten, stereoskopische Abbildung eines Operationsfeldes. In der Reihenfolge vom betrachteten Objekt aus gesehen sind einige wesentliche Komponenten:
- eine Frontlinse zum Abbilden eines Objekts, z.B. eines Patientenauges in der Objektebene der Frontlinse,
- ein Vergrößerungswechsler, z.B. ein Galilei-System oder ein optischer Zoom, um eine gewünschte Vergrößerung einzustellen und dem Einblick das vergrößerte Bild zuzuleiten, und
- ein Einblick mit zwei Okularen, in den der Chirurg während einer Operation blickt, um das Operationsfeld stereoskopisch zu beobachten.

Zwischen Frontlinse und Einblick können weitere optische Zubehörkomponenten eingefügt sein, z.B. ein Strahlenteiler, der einen Teil des Lichtes auf eine Videokamera lenkt. Außerdem enthalten Operationsmikroskope eine Beleuchtung, die das Operationsfeld durch die Frontlinse hindurch beleuchtet.

Ein Operationsmikroskop besitzt jeweils zwei separate Beobachtungsstrahlengänge für jeden Beobachter: Durch den Versatz des linken Beobachtungsstrahlengangs, der zum linken Okular, und des rechten Beobachtungsstrahlengangs, der zum rechten Okular führt, um die sogenannte Stereobasis entstehen im linken und rechten Okular des Operationsmikroskops Bilder desselben Objekts, jedoch aus leicht unterschiedlichen Perspektiven, die bei gleichzeitiger Betrachtung zu einem stereoskopischen Bild fusionieren. Alle Beobachtungsstrahlengänge verlaufen üblicherweise durch eine/die gemeinsame Frontlinse hindurch.

Mit einem derartigen Operationsmikroskop, dessen Frontlinse häufig eine Brennweite von 175mm oder 200mm besitzt, kann der Chirurg den vorderen Abschnitt eines Patientenauges stereoskopisch beobachten. Um jedoch den Augenhintergrund zu beobachten, ist ein zusätzliches optisches Modul erforderlich, das in den optischen Strahlengang des Mikroskops zwischen das beobachtete Patientenauge und das Operationsmikroskop eingefügt werden muss. Dies ist begründet in der hohen Brechkraft der Cornea und der Augenlinse.

Für eine Weitwinkel-Fundusbeobachtung, d.h. um einen großen Bereich des Augenhintergrunds beobachten zu können, enthalten derartige Module als wesentlichen Bestandteil eine sogenannte Ophthalmoskopierlinse mit kurzer Brennweite (z.B. f = 10 mm), um außerhalb des Auges eine Abbildung des Augenhintergrundes zu erzeugen. Diese Abbildung kann nun mit dem Operationsmikroskop beobachtet werden.

Weil die Abbildung des Augenhintergrunds höhen- und seitenvertauscht ist, muss diese mit einer weiteren Optik erneut in Höhe und Seite vertauscht werden, bevor sie in den Okularen dargestellt wird. Ansonsten ist eine Hand-Auge Koordination für den Chirurgen nicht gegeben. Prinzipiell kann diese weitere Optik zur Bildaufrichtung entweder mit der Ophthalmoskopierlinse in einer Einheit vor der Frontlinse des Operationsmikroskops zusammengefasst werden, oder die weitere Optik zur Bildaufrichtung wird an geeigneter Stelle in das Operationsmikroskop eingefügt.

Das Zusammenfassen der erforderlichen Optiken in einer Einheit vor der Frontlinse besitzt den Vorteil, sehr kosteneffizient zu sein und die Bauhöhe des Mikroskopkörpers nicht zu vergrößern. Diese Einheit kann schnell an ein Mikroskop montiert und wieder demontiert werden und kann daher mit geringem Aufwand auch abwechselnd mit mehreren Operationsmikroskopen genutzt werden.

Diese Erfindung bezieht sich auf ein Weitwinkel Fundusbeobachtungssystem, bei dem die Ophthalmoskopierlinse und die Optik zur Bildaufrichtung in einer Einheit zwischen Patientenauge und Operationsmikroskop zusammengefasst sind. Dieses System wird an einem Drehlager montiert, mit dem es um die Achse des Drehlagers gedreht werden kann.

Durch die Drehung um dessen Drehachse kann das Modul so positioniert werden, dass es den Chirurgen bei der Instrumentenführung nicht behindert und andererseits auch den Patienten nicht berührt, z.B. dessen Nase. Dies würde bei Bewegung des Patienten zu Bildunschärfen führen.

Dieses Weitwinkel Fundusbeobachtungssystem kann nach Bedarf in den optischen Strahlengang des Mikroskops zwischen Frontlinse und Patientenauge hinein- und herausgeschwenkt werden.

Die optischen Weglängen in dem Weitwinkel Fundusbeobachtungssystem sind so ausgelegt, dass nach dem Einschwenken des Systems unter die Frontlinse in den optischen Strahlengang des Mikroskops ein scharfes Bild des Augenhintergrunds in den Okularen dargestellt wird.

Bei bisherigen Systemen führt ein Drehen des Gehäuses mit dem Drehlager dazu, dass das im Okular sichtbare zunächst zentrierte Bild des Augenhintergrunds aus der Mitte des O-kularbildes zur Seite hin auswandert. Dies ist insbesondere bei höheren Vergrößerungsfaktoren des Mikroskops sehr störend, weil dann große Teile abgeschnitten werden und im Okular nicht mehr sichtbar sind.

Die Aufgabe der Erfindung besteht darin, ein Modul der eingangs genannten Art zu schaffen, bei dem dieser Nachteil vermieden wird.

Die Lösung besteht darin, dass das Prismensystem so angeordnet ist, dass ein am Ort des Zwischenbildes entlang der optischen Achse der Opthalmoskopierlinse verlaufender Lichtstrahl nach Austritt aus dem Zusatzmodul mit der Drehachse des Drehlagers zusammenfällt.

Bei einer vorteilhaften Ausführungsform ist vorgesehen, dass eines der Prismen des Prismensystems senkrecht zur optischen Achse der Ophthalmoskopierlinse verschiebbar ist, um die optische Weglänge des Prismensystems einzustellen.

Dadurch verlängert oder verkürzt sich der optische Weg, den vom Zwischenbild ausgehende Lichtstrahlen in dem Modul zurücklegen, und eine Fehlsichtigkeit des Patientenauges kann ausgeglichen werden.

Eine vorteilhafte Ausführungsform zeichnet sich dadurch aus, dass das Modul mit einer Führung und Drehknöpfen versehen ist, durch deren Drehung das eine Prisma verschoben werden kann.

Zweckmäßigerweise ist dabei vorgesehen, dass die Drehknöpfe in eine mit dem Gehäuse abschließenden Welle eingesteckt und abgezogen werden können.

Insbesondere kann dabei vorgesehen sein, dass die Drehknöpfe auf beiden Seiten des Gehäuses einsteckbar sind. Zweckmäßigerweise ist das Gehäuse an einer Aufhängung mit einer Linearführung befestigt. Dadurch kann das Gehäuse aus Sicherheitsgründen in Richtung der Frontlinse nach oben ausweichen, falls der Patient dagegen stößt.

Die Erfindung wird im Folgenden anhand einer vorteilhaften Ausführungsform unter Bezugnahme auf die beigefügten Zeichnungen beispielsweise beschrieben. Es zeigen:
- Fig. 1: das Weitwinkel Fundus-Beobachtungssystem der Erfin- dung im Schnitt;
- Fig. 2: eine Mikroskopansicht von vorne (aus Sicht des Chi- rurgen) mit sterilen Kappen und steriler Hülle;
- Fig. 3: die Frontlinse und das Vergrößerungssystem im Schnitt mit linkem und rechtem Strahlengang sowie die Stereobasis;
- Fig. 4: eine Seitenansicht des Mikroskops, wobei das Weit- winkel Fundusbeobachtungssystem aus dem Strahlen- gang des Operationsmikroskops herausgeschwenkt ist,
- Fig. 5: eine Seitenansicht des Mikroskops mit steriler Hülle für das Gehäuse;
- Fig. 6: das Bild des Augenhintergrunds im Okular, zentriert
- Fig. 7: das Bild des Augenhintergrunds im Okular, de- zentriert
- Fig. 8: eine Detailansicht der Linsenfassung mit L-förmigen Ausfräsungen und Zylinderstiften der Linsenaufnahme im Gehäuse; und
- Fig. 9: die Schwalbenschwanzverbindung im Detail.

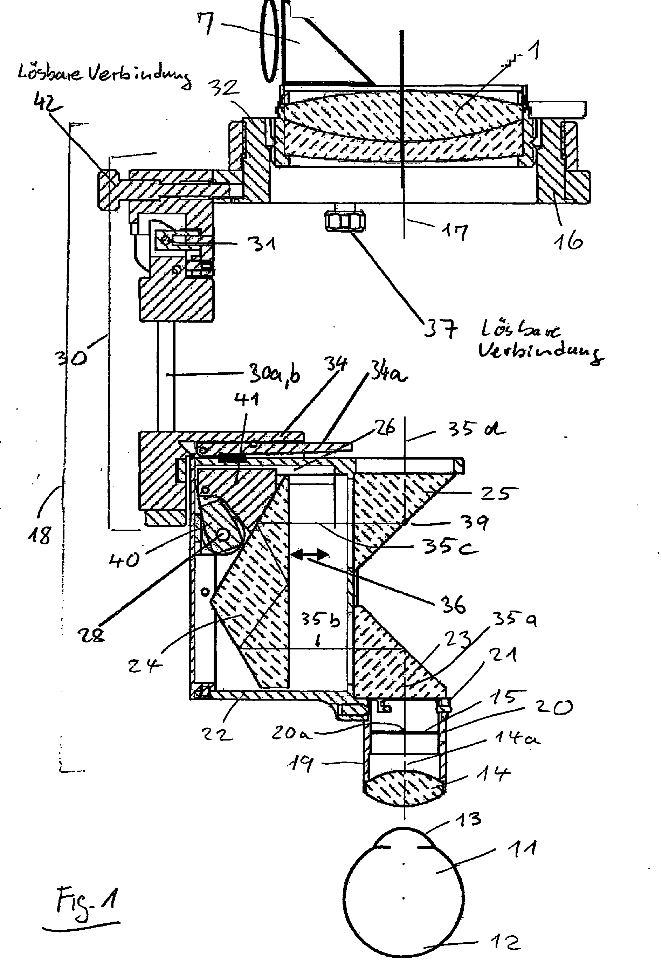

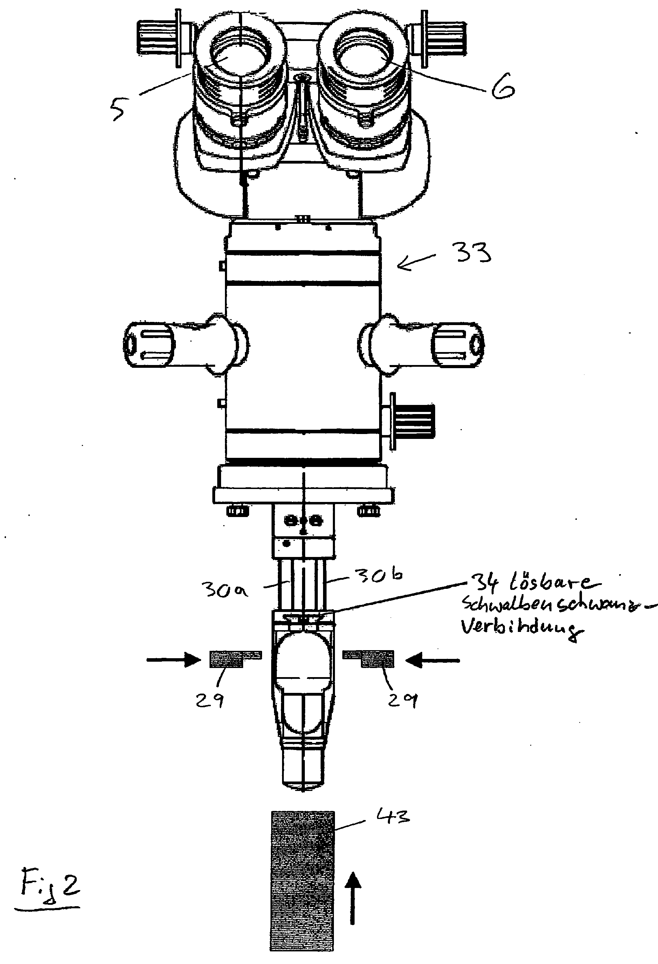

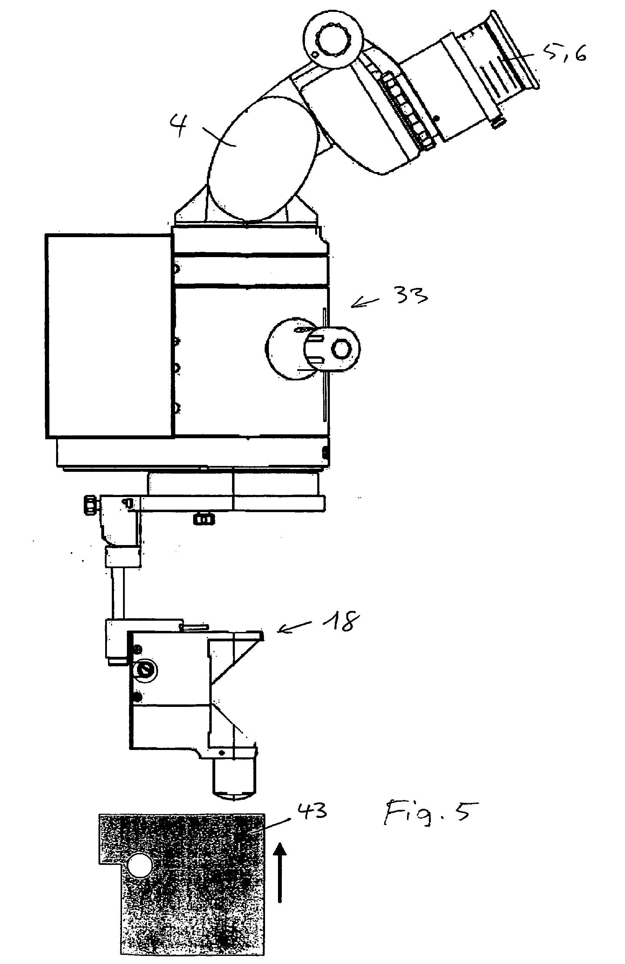

Wie dies in Fig. 1 gezeigt ist, ist das erfindungsgemäße Modul (18) vor der Frontlinse 1 eines Operationsmikroskops angeordnet. Es setzt sich im Wesentlichen aus den folgenden Bestandteilen zusammen:
- einer Ophthalmoskopierlinse 14 mit einer optischen Achse 14a in einer Linsenfassung 19 zur Erzeugung einer Abbildung (genannt Zwischenbild) 15 des Augenhintergrunds 12 eines Patientenauges 11 am Ort einer Bildfeldblende 20,
- einer runden Bildfeldblende 20 an der Linsenfassung 19 am Ort des Zwischenbildes 15 mit einem Mittelpunkt 20a,
- einer Aufnahme 21 für die Linsenfassung 19 am Gehäuse 22,
- einem Gehäuse 22 zum Aufnehmen und Halten der optischen Komponenten 14, 23, 24, 25,
- einer Prismenanordnung, bestehend aus zwei 90°-Prismen 23,25 und einem Bauernfeindprisma 24 mit Dachkante zur optischen Aufrichtung des Zwischenbilds 15,
- einer Linearführung 26 und einer Verstellmechanik 26, 28, 40, 41, um das Bauernfeindprisma 24 mit Dachkante senkrecht zur optischen Achse 14a der Ophthalmoskopierlinse 14 in Richtung des Doppelpfeils 36 zu verschieben
- einer Welle 28 mit exzentrischem Eingriff in die Linearführung 26, um das Bauernfeindprisma mit Dachkante 24 durch Drehen der Welle entlang der Linearführung zu verschieben,
- Drehknöpfen 29 (Fig. 2) zum Einstecken in die Welle 28,
- einer Aufhängung 30 für das Gehäuse 22 mit einer Linearführung 30a, 30b,
- einem Schwenkgelenk 31 zum Ein- und Ausschwenken des Gehäuses 22 und der Aufhängung 30,
- einem Drehlager 16, um das Gehäuse 22 um die Drehachse 17 zu drehen,
- einer mit dem Drehlager kombinierten Mikroskopaufnahme 32, um das Weitwinkel Fundusbeobachtungssystem 18 an ein Operationsmikroskop 33 (Fig. 2) zu montieren, so dass das Drehlager im Bereich der Frontlinse (1) des Operationsmikroskops 33 sitzt,
- lösbaren Verbindungen 34 zwischen Aufhängung 30 und Gehäuse 22,
- einer lösbaren Verbindung 42 zwischen Aufhängung 30 und Drehlager 16,
- einer lösbaren Verbindung 37 zwischen Mikroskopaufnahme 32 und Mikroskop 33, und
- einer Hülle 43 (Fig. 2 und 5) für das Gehäuse 22, die steril aufbereitbär ist und die zur Vermeidung eine Kontamination vor der Operation über das Gehäuse 22 geschoben wird.

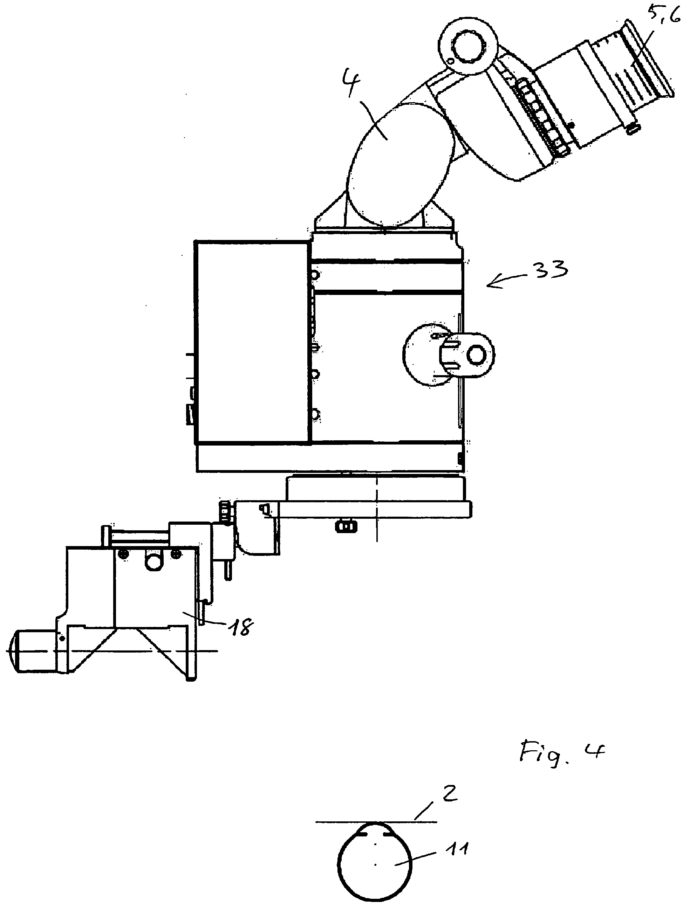

In Fig. 1 ist noch ein Prisma 7 gezeigt, miot dem die Mikroskopbeleuchtung durch die Frontlinse 1 hindurch zur Ojektebene 2 (Fig. 4) gelenkt wird. Fig. 2 zeigt eine Gesamtansicht von Mikroskop mit dem daran angebrachten erfindungsgemäßen Modul 18.

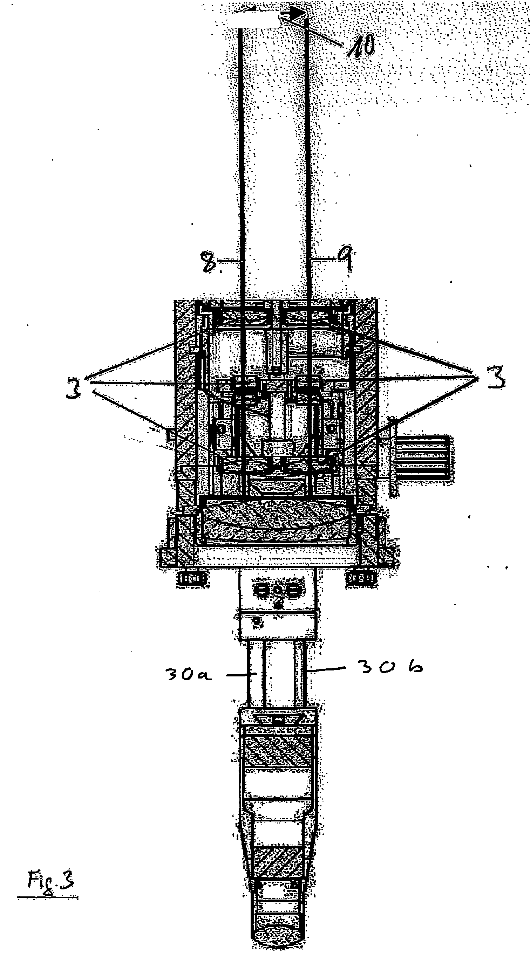

In Fig. 3 ist ein Vergrößerungswechsler 3 gezeigt, der z.B. ein Galilei-System oder ein optischer Zoom sein kann, um eine gewünschte Vergrößerung einzustellen und dem Einblick 5, 6 das vergrößerte Bild zuzuleiten. Weiter sind dort die zwei separaten Beobachtungsstrahlengänge 8, 9 gezeigt.

Durch den Versatz des linken Beobachtungsstrahlengangs 8, der zum linken Okular 5, und des rechten Beobachtungsstrahlengangs 9, der zum rechten Okular 6 führt, um die sogenannte Stereobasis 10 entstehen im linken und rechten Okular 5, 6 des Operationsmikroskops 33 Bilder desselben Objekts, die das stereoskopische Bild ergeben. Alle Beobachtungsstrahlengänge verlaufen üblicherweise durch eine/die gemeinsame Frontlinse 1 hindurch. Alle Beobachtungsstrahlengänge verlaufen auch gemeinsam durch die Prismenanordnung 25, 24, 23 hindurch und treffen sich im zwischenbild 20.

In Fig. 4 und 5 ist das System von der Seite gezeigt. Man erkennt den Einblick 4 mit den beiden Okularen 5, 6 und der Objektebene 2 des Mikroskops 33. Das erfindungsgemäße Modul 18 ist in Fig. 4 aus dem Abbildungsstrahlengang heraus und in Fig. 5 in denselben hineingeschwenkt.

Die Opthalmoskopierlinse 14 des Weitwinkel Fundusbeobachtungssystem 18 befindet sich häufig ca. 4 - 9 mm über der Cornea 13 des Patientenauges 11. Sinnvollerweise ist diese Linse mittig zum Auge angeordnet. Diese Linse ist in eine Fassung 19 eingeklebt. Die Linse erzeugt eine Abbildung (Zwischenbild) 15 des Augenhintergrunds 12 am Ort der Bildfeldblende 20. Die Ophthalmoskopierlinse 14 besitzt eine optische Achse 14a, die auch durch den Mittelpunkt 20a der Bildfeldblende 20 verläuft.

Das Zwischenbild entsteht bei Fehlsichtigkeit des Patientenauges vor oder hinter der Bildfeldblende 20. Dies kann durch Verschieben des Bauernfeindprismas mit Dachkante 24 ausgeglichen werden, sodass in den Okularen 5, 6 (Fig. 2) wieder ein scharfes Bild des Augenhintergrunds dargestellt wird.

Die von dem Zwischenbild ausgehenden Lichtstrahlen werden von dem Prismensystem, bestehend aus den drei Prismen 23, 24, 25 umgelenkt. Ein auf der optischen Achse 14a der Ophthalmoskopierlinse 14 verlaufender Lichtstrahl 35a, 35b, 35c, 35d wird von dem ersten Prisma 23 unter 90° abgelenkt und auf das Bauernfeindprisma mit einer Dachkante (24) gelenkt.

Dieser aus dem Prisma 24 wieder austretende Lichtstrahl 35c verläuft parallel zum eintretenden Lichtstrahl 35b und wird von dem dritten Prisma 25 um 90° in Richtung der Frontlinse 1 des Operationsmikroskops 33 umgelenkt (Lichtstrahl 35d).

Das Bauernfeindprisma mit einer Dachkante 24 ist seitlich in der von dem Doppelpfeil 36 angedeuteten Richtung verschiebbar. Dadurch verlängert oder verkürzt sich der optische Weg, den vom zwischenbild ausgehende Lichtstrahlen in dem System zurücklegen, und eine Fehlsichtigkeit des Patientenauges kann ausgeglichen werden. Der Chirurg kann dazu durch Drehen der Welle 28 mit einem Knopf 29 das Bauernfeindprisma mit Dachkante 24 in der von dem Doppelpfeil 36 angedeuteten Richtung verschieben.

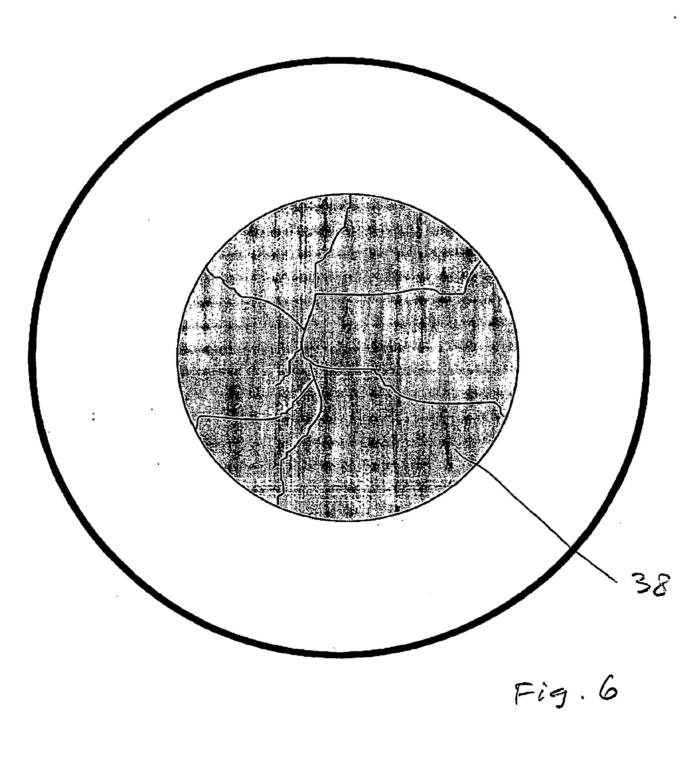

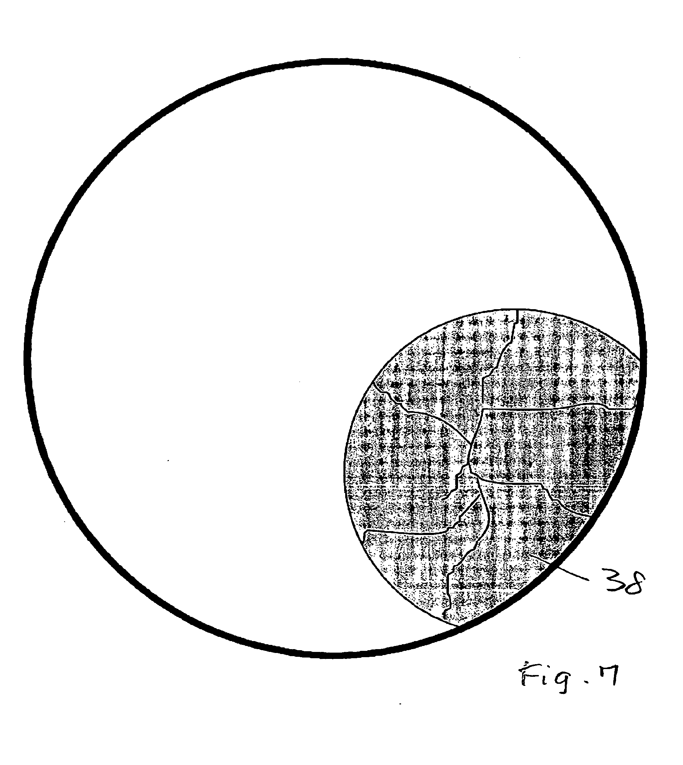

Die Abbildung 38 (Fig. 6 und 7) des Augenhintergrunds 12 stellt sich in beiden Okularen 5, 6 als kreisrundes, zentriertes Bild dar. Weil die Prismenanordnung 23, 24, 25 mit dem Gehäuse 22 nahe dem Patientenauge positioniert wird, können sowohl der rechte als auch der linke Beobachtungsstrahlengang durch das System hindurch bis zum Zwischenbild 15 verlaufen, ohne abgedeckt zu werden. Das zwischenbild 15 kann also im Einblick beim Blick durch die Okulare stereoskopisch beobachtet werden. Die jeweiligen rechten und linken Beobachtungsstrahlengänge verlaufen unter einem Winkel zueinander durch die Prismen 25, 24, 23 und treffen sich im Zwischenbild 15.

Das Gehäuse ist an einer Aufhängung (30) mit einer Linearführung 30a, 30b befestigt. Dadurch kann das Gehäuse 22 aus Sicherheitsgründen in Richtung der Frontlinse 1 nach oben ausweichen, falls der Patient dagegen stößt.

Wenn das Modul 18 um die Drehachse 17 des Drehlagers 16 gedreht wird, besteht die Gefahr des Auswanderns des Bildes 38 des Augenhintergrunds im Okular von der in Fig. 6 gezeigten Stellung z.B. in die in Fig. 7 gezeigte Stellung. Dieser Mangel kann durch das Auffinden einer Bedingung beseitigt werden, deren Einhaltung garantiert, dass das Bild des Augenhintergrunds auch bei Drehungen ortsfest im Okular erscheint. Demzufolge muss erreicht werden, dass ein Lichtstrahl 35a, 35b, 35c, 35d, der auf der optischen Achse 14a der Ophthalmoskopierlinse 14 durch die Mitte des Zwischenbildes 20a hindurch verläuft, nach Durchlaufen des Prismensystems 23, 24, 25 koinzident mit der Drehachse 17 des Drehlagers verläuft.

Denn unter dieser Bedingung wird erreicht, dass der Lichtstrahl, der in den Okularen 5, 6 in den Mittelpunkt des Fundusbildes 38 abgebildet wird, durch die Drehung keiner Veränderung seines Verlaufs unterliegt. Zur Einhaltung der Bedingung ist das Prisma 25 so ausgelegt, dass der Lichtstrahl 35c am Durchstoßungspunkt 39 der Drehachse 17 durch die reflektierende Fläche des Prismas 25 umgelenkt wird.

Ein wesentlicher Gesichtspunkt betrifft die Knöpfe 29 zum Drehen der Welle 28. Der auf der Welle 28 befestigte Mitnehmer 40 verschiebt beim Drehen der Welle 28 das Prisma 24 auf einem Reiter 41 entlang der Linearführung 26.

Die Fokussierung wird intraoperativ mit den sterilen Drehknöpfen 29 eingestellt. Hier ist jedoch zu bedenken, dass das Gehäuse 22 aufgrund der enthaltenen, empfindlichen Optiken nicht autoklaviert werden kann. Stattdessen kann es erfindungsgemäß mit der sterilisierten Hülle 43 versehen werden. Diese Hülle, die vorteilhafterweise einteilig ist, muss ergonomisch über das Gehäuse 22 geschoben werden können. Es dürfen daher keine aus dem Gehäuse hervorragenden Knöpfe vorhanden sein.

Beide Anforderungen können erfindungsgemäß dadurch erfüllt werden, dass die Drehknöpfe 29 von der Welle 28 abnehmbar sind und die Welle 28 mit dem Gehäuse 22 bündig abschließt. Dazu wird die Welle mit einer halbkreisförmigen Einsenkung versehen, in die der Drehknopf 29 mit einer entsprechenden halbkreisförmigen Ausbuchtung formschlüssig eingesteckt werden kann. Die Drehknöpfe werden zum Aufziehen der sterilen Hülle 43 abgenommen und danach in die Welle 28 eingesteckt.

Für die Ophthalmoskopierlinse 14 besteht die Anforderung, dass sie mitsamt Fassung zum Sterilisieren vom Gehäuse entfernt und danach wieder angebracht werden soll. Übliche Schraubgewinde, z.B. Feingewinde, sind sehr unkomfortabel. Die Gewinde verkanten leicht, sind aufgrund des Aufbereitungsverfahrens fettfrei und deshalb schlecht zu schrauben und erfordern beim Einschrauben ein häufiges Umgreifen.

Die Linsenfassung 19 sollte mit einer Hand durch Drehen verriegelbar sein, ohne dass die ausführende Person umgreifen muss. Versuche ergaben, dass der Drehwinkel idealerweise kleiner als 20° sein sollte.

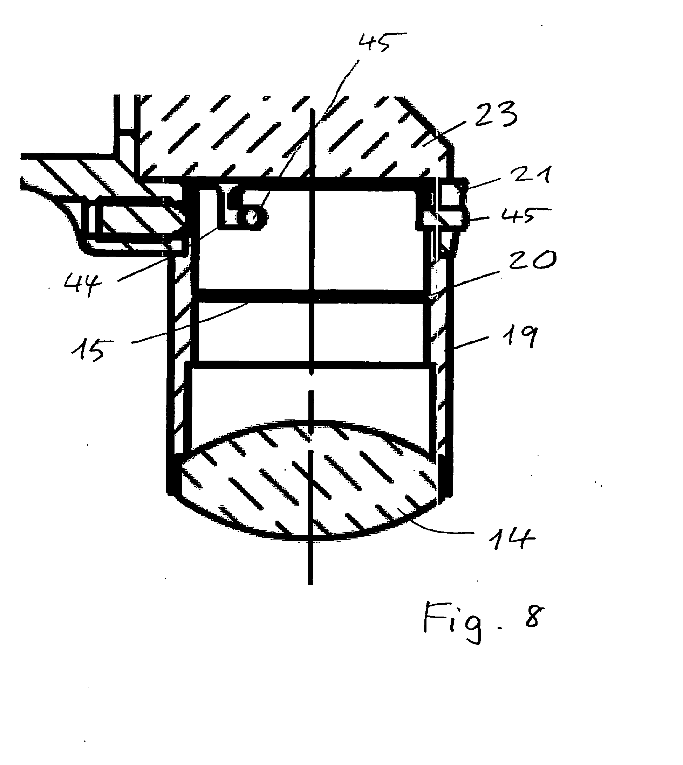

Die schnelle und ergonomische Befestigung der Ophthalmoskopierlinse 14 am Gehäuse soll nun im Zusammenhang mit Fig. 8 beschrieben werden. Die Befestigung wird durch eine besondere Ausformung der Linsenfassung 19 erreicht, die jeweils drei Ausfräsungen 44 in Form eines "L" besitzt. Die Linsenfassung wird von unten in das Gehäuse 22 eingesteckt, sodass drei im Gehäuse an der Linsenaufnahme angebrachte Zylinderstifte 45 in diese Ausfräsungen 44 eingreifen. Durch ein Drehen der Linsenfassung um weniger als 20° Grad (aus dem Handgelenk) greifen diese Stifte in den horizontalen Bereich der Ausfräsungen 44 ein, und die Linsenfassung 19 ist verriegelt.

Um die Berührung von unsterilen Teilen während der Operation weitgehend zu vermeiden, sind das Drehlager 16, die Aufhängung 30, die Drehknöpfe 29, die Ophthalmoskopierlinse 14 mit Fassung 19 und die Hülle 43 für das Gehäuse 22 in einem Dampfautoklavieren bei 134°C und 3 bar Druck sterilisierbar ausgeführt.

Dazu ist es erforderlich, dass die Komponenten mit schnell zu lösenden Verschlüssen ohne Werkzeug miteinander verbunden werden.

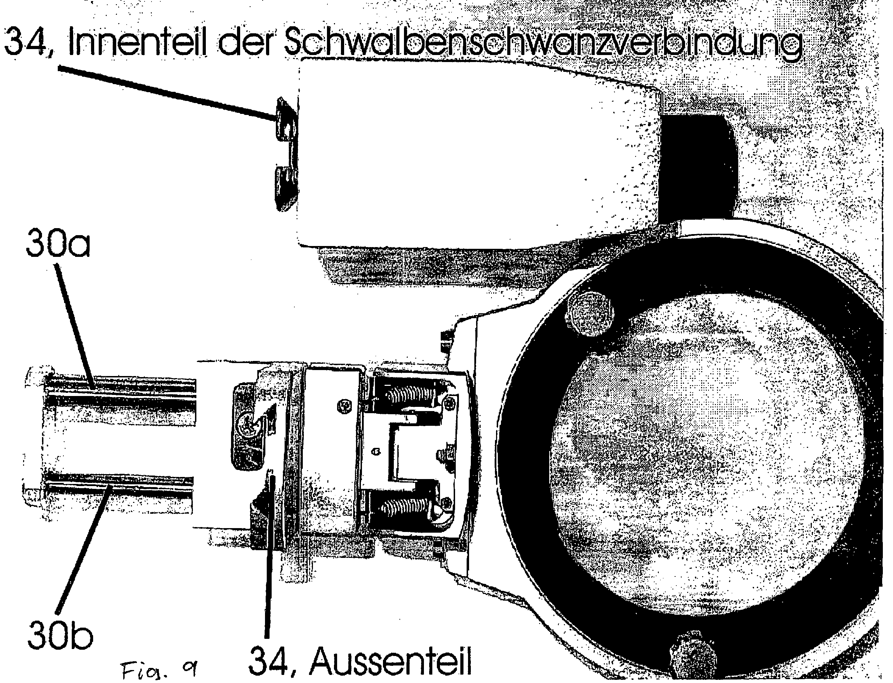

Insbesondere muss das Ansetzen des Gehäuses 22 an die Aufhängung 30 möglich sein, ohne dass eine Person, die das unsterile Gehäuse 22 berührt, dazu auch die sterile Aufhängung 30 berühren muss. Diese Anforderung wird dadurch erfüllt, dass diese Verbindung 34 als Schwalbenschwanzverbindung ausgeführt ist, und dass das Gehäuse 22 einen federnden Verriegelungshebel 34a aufweist, bei dessen Niederdrücken die Schwalbenschwanzverbindung freigegeben wird. Eine Person kann auf diese Weise das Gehäuse 22 mit einer Hand festhalten, gleichzeitig den Verriegelungshebel 34a niederdrücken und die Schwalbe des Gehäuses 22 in das Gegenstück an der sterilen Aufhängung 30 einschieben, ohne dass die Person die sterile Aufhängung 30 berührt. Die Schwalbenschwanzverbindung ist in Fig. 9 gezeigt.

## Patentansprüche

1. Modul (18) für die stereoskopische Weitwinkel-Fundusbeobachtung mit einem Operationsmikroskop (33) der Augenchirurgie zum Positionieren zwischen einem Patientenauge (11) und der Frontlinse (1) des Operationsmikroskops (33),
mit einer Opthalmoskopierlinse (14) mit einer optischen Achse (14a) zur Abbildung des Fundus (12) in ein Zwischenbild (15),
mit einer Prismenanordnung (23, 24, 25) zur optischen Aufrichtung des Zwischenbildes (15),
mit einem Gehäuse (22), das die Anordnung aus Ophthalmoskopierlinse (14) und Prismenanordnung (23, 24, 25) enthält, und
mit einer Vorrichtung zum Montieren an ein Operationsmikroskop
mit einem Drehlager (16) zum Drehen des Moduls (18) um die Achse des Drehlagers (17) und einer Aufhängung (30) für das Mikroskop (33),
**dadurch gekennzeichnet, dass**
das Prismensystem (23, 24, 25) so angeordnet ist, dass ein am Ort des Zwischenbildes (15) entlang der optischen Achse (14a) der Opthalmoskopierlinse (14) verlaufender Lichtstrahl (35a) nach Austritt aus dem Modul (18) mit der Drehachse (17) des Drehlagers (16) zusammenfällt.

2. Modul nach Anspruch 1, **dadurch gekennzeichnet, dass** eines der Prismen (24) des Prismensystems (23, 24, 25) senkrecht zur optischen Achse (14a) der Ophthalmoskopierlinse (14) verschiebbar ist, um die optische Weglänge des Prismensystems (23, 24, 25) einzustellen.

3. Modul nach Anspruch 2, **dadurch gekennzeichnet, dass** es mit einer Führung und Drehknöpfen (29) versehen ist, durch deren Drehung das eine Prisma (24) verschoben werden kann,

4. Modul nach Anspruch 3, **dadurch gekennzeichnet, dass** die Drehknöpfe (29) in eine mit dem Gehäuse (22) bündig abschließende Welle (28) eingesteckt und abgezogen werden können.

5. Modul nach Anspruch 4, **dadurch gekennzeichnet, dass** die Drehknöpfe (29) auf beiden Seiten des Gehäuses (22) einsteckbar sind.

6. Modul nach einem der Ansprüche 1 - 5, **dadurch gekennzeichnet, dass** die Ophthalmoskopierlinse (14) in einer Linsenfassung (19) aufgenommen ist, die durch Drehen der Linsenfassung (19) um ihre Achse (14a) um weniger als 20° vom Gehäuse (22) lösbar oder daran befestigbar ist.

7. Modul nach einem der Ansprüche 1 - 6, **dadurch gekennzeichnet, dass** Drehlager (16), Aufhängung (30), der Drehknopf (29) und die Ophthalmoskopierlinse (14) in einem Dampfautoklaven autoklavierbar sind.

8. Modul nach einem der Ansprüche 1 - 6, **dadurch gekennzeichnet, dass** die Opthalmoskopierlinse (14) mit Fassung (19), die Drehknöpfe (29), die Aufhängung (30) und das Drehlager (16) in einem Dampfautoklaven bei 134°C autoklavierbar ausgeführt sind.

9. Modul nach einem der Ansprüche 1 - 8, **dadurch gekennzeichnet, dass** das Gehäuse (22) an einer Aufhängung (30) mit einer Linearführung (30 a, b) befestigt ist.

10. Modul nach einem der Ansprüche 1 - 9, **dadurch gekennzeichnet, dass** die Verbindung zwischen Gehäuse (22) und Aufhängung (30) eine Schwalbenschwanzverbindung (34) aufweist, die durch Niederdrücken eines Hebels (34a) lösbar und durch Freigabe des Hebels (34a) verriegelbar ist.

## Claims

1. Module (18) for stereoscopic wide-angle fundus observation with a surgical microscope (33) used in ophthalmic surgery, to be positioned between a patient's eye (11) and the front lens (1) of the surgical microscope (33),
with an ophthalmoscopic lens (14) having an optical axis (14a) for imaging the fundus (12) into an intermediate image (15),
with a prism arrangement (23, 24, 25) for optical erection of the intermediate image (15),
with a housing (22) which contains the arrangement consisting of ophthalmoscopic lens (14) and prism arrangement (23, 24, 25), and
with a device for mounting onto a surgical microscope, with a pivot bearing (16) for rotating the module (18) about the axis (17) of the pivot bearing and with a suspension (30) for the microscope (33),
**characterised in that**
the prism system (23, 24, 25) has been arranged in such a way that a light beam (35a) running along the optical axis (14a) of the ophthalmoscopic lens (14) at the location of the intermediate image (15) coincides with the axis of rotation (17) of the pivot bearing (16) after emerging from the module (18).

2. Module according to Claim 1, **characterised in that** one of the prisms (24) of the prism system (23, 24, 25) is displaceable perpendicularly to the optical axis (14a) of the ophthalmoscopic lens (14) in order to adjust the optical path length of the prism system (23, 24, 25).

3. Module according to Claim 2, **characterised in that** it has been provided with a guide and with rotary knobs (29), by rotation of which one prism (24) can be displaced.

4. Module according to Claim 3, **characterised in that** the rotary knobs (29) can be inserted into and removed from a shaft (28) terminating flush with the housing (22).

5. Module according to Claim 4, **characterised in that** the rotary knobs (29) are capable of being inserted on both sides of the housing (22).

6. Module according to one of Claims 1 - 5, **characterised in that** the ophthalmoscopic lens (14) is received in a lens mount (19) which by rotating the lens mount (19) about its axis (14a) by less than 20° is capable of being detached from the housing (22) or of being fastened thereto.

7. Module according to one of Claims 1 - 6, **characterised in that** the pivot bearing (16), the suspension (30), the rotary knob (29) and the ophthalmoscopic lens (14) are autoclavable in a steam autoclave.

8. Module according to one of Claims 1 - 6, **characterised in that** the ophthalmoscopic lens (14) with mount (19), the rotary knobs (29), the suspension (30) and the pivot bearing (16) are designed to be autoclavable in a steam autoclave at 134 °C.

9. Module according to one of Claims 1 - 8, **characterised in that** the housing (22) has been fastened to a suspension (30) with a linear guide (30 a, b).

10. Module according to one of Claims 1 - 9, **characterised in that** the connection between the housing (22) and the suspension (30) exhibits a dovetail joint (34) which is separable by depressing a lever (34a) and lockable by releasing the lever (34a).

## Revendications

1. Module (18) d'observation ophtalmoscopique à grand angle stéréoscopique avec un microscope d'opération (33) pour la chirurgie ophtalmologique, destiné à être positionné entre l'oeil (11) d'un patient et la lentille frontale (1) du microscope d'opération (33),
comportant une lentille ophtalmoscopique (14) avec un axe optique (14a) pour reproduire le fond d'oeil (12) dans une image intermédiaire (15),
comportant un système de prismes (23, 24, 25) pour le redressement optique de l'image intermédiaire (15),
comportant un boîtier (22), qui contient le système formé par la lentille ophtalmoscopique (14) et le système de prismes (23, 24, 25), et
comportant un dispositif pour le montage sur un microscope d'opération,
comportant un palier rotatif (16) pour faire tourner le module (18) autour de l'axe du palier rotatif (16) et une suspension (30) pour le microscope (33),
**caractérisé en ce que**
le système de prismes (23, 24, 25) est agencé de telle sorte qu'un rayon lumineux (35a), s'étendant sur le lieu de l'image intermédiaire (15) le long de l'axe optique (14a) de la lentille ophtalmoscopique (14), coïncide, après la sortie hors du module (18), avec l'axe de rotation (17) du palier rotatif (16).

2. Module selon la revendication 1, **caractérisé en ce qu'**un des prismes (24) du système de prismes (23, 24, 25) peut être déplacé perpendiculairement à l'axe optique (14a) de la lentille ophtalmoscopique (14), afin de régler la longueur de trajectoire optique du système de prismes (23, 24, 25).

3. Module selon la revendication 2, **caractérisé en ce qu'**il est muni d'un guidage et de boutons de réglage (29), dont la rotation permet de déplacer un prisme (24).

4. Module selon la revendication 3, **caractérisé en ce que** les boutons de réglage (29) peuvent être enfichés dans un axe (28) situé à fleur du boîtier (22) et peuvent en être retirés.

5. Module selon la revendication 4, **caractérisé en ce que** les boutons de réglage (29) peuvent être enfichés sur les deux côtés du boîtier (22).

6. Module selon l'une quelconque des revendications 1 à 5, **caractérisé en ce que** la lentille ophtalmoscopique (14) est logée dans une monture (19) et, par une rotation de moins de 20° de la monture (19) autour de son axe (14a), peut être désolidarisée du boîtier (22) ou être fixée contre celui-ci.

7. Module selon l'une quelconque des revendications 1 à 6, **caractérisé en ce que** le palier rotatif (16), la suspension (30), le bouton de réglage (29) et la lentille ophtalmoscopique (14) peuvent être traités dans un autoclave à vapeur.

8. Module selon l'une quelconque des revendications 1 à 6, **caractérisé en ce que** la lentille ophtalmoscopique (14) avec la monture (19), les boutons de réglage (29), la suspension (30) et le palier rotatif (16) sont réalisés de manière à pouvoir être traités à 134°C dans un autoclave à vapeur.

9. Module selon l'une quelconque des revendications 1 à 8, **caractérisé en ce que** le boîtier (22) est fixé à une suspension (30) avec un guidage linéaire (30a, b).

10. Module selon l'une quelconque des revendications 1 à 9, **caractérisé en ce que** l'assemblage entre le boîtier (22) et la suspension (30) est un assemblage en queue d'aronde (34), qui peut être désolidarisé par l'abaissement d'un levier (34a) et peut être verrouillé par le déblocage du levier (34a).
